# FASCICULE DE BREVET EUROPEEN

(11) **EP 1 661 589 B1**
(45) Date de publication et mention de la délivrance du brevet: **10.06.2009**
(21) Numéro de dépôt: 05292473.5
(22) Date de dépôt: 22.11.2005
(51) Int. Cl.: A61B 5/15, A61M 1/02

(54) **Système à poches pour l'échantillonnage automatique d'un fluide biologique**
Beutelanordnung zum automatischen Entnehmen von biologischen Fluidproben
Pouch system for automatic sampling of a biological fluid

(30) Priorité: 24.11.2004 FR 0452749
(43) Date de publication de la demande: 31.05.2006
(73) Titulaire: MACO PHARMA, 59420 Mouvaux (FR)
(72) Inventeur: Goudaliez, Francis, 59155 Faches-Thumesnil (FR)
(74) Mandataire: Breese, Pierre

(56) Documents cités:
- FR-A- 2 850 581
- US-A- 6 113 554
- US-A1- 2001 025 167
- US-A1- 2002 068 675
- US-A1- 2003 062 318

## Description

L'invention concerne un système à poches destiné au prélèvement et à l'échantillonnage d'un fluide biologique, ainsi qu'un ensemble comprenant une pompe péristaltique et un tel système à poches.

Elle s'applique typiquement au cas où le fluide biologique est du sang total qui doit être prélevé d'un donneur dans une poche de recueil en étant additionné d'une solution anticoagulante et/ou de conservation. Lors d'un prélèvement de sang, un échantillon du sang prélevé doit être analysé, notamment afin de déterminer le groupe Rhésus, de réaliser une numération et de détecter d'éventuelles contaminations comme des virus, des bactéries ou d'autres éléments étrangers au sang du donneur.

Pour réaliser l'échantillonnage, on peut utiliser un système à poches tel que décrit dans le document FR-A-2 851 167. Un tel système à poches comprend un moyen de prélèvement du sang qui est en communication fluidique avec au moins une poche de recueil du sang. Le système comprend en outre une poche d'échantillonnage destinée à recueillir les premiers millilitres de sang prélevé, et des tubes d'échantillonnage du sang associés au système à poches par l'intermédiaire d'un corps porte tube. Lors de l'échantillonnage, l'utilisateur remplit les tubes avec une partie du sang contenu dans la poche d'échantillonnage en poussant successivement les tubes dans le corps porte tube.

Le document FR-A-2 850 581 décrit un système a poches destiné au prélèvement et à l'échantillonnage d'un fluide biologique, notamment sanguin, du type comprenant un moyen de prélèvement du dit fluide, au moins une poche contenant une solution anticoagulante et/ou de conservation du fluide prélevé, au moins une poche de recueil destinée a recevoir le fluide prélevé additionné de la solution anticoagulante et/ou de conservation, et un dispositif d'échantillonnage du fluide prélevé, dans lequel le moyen de prélèvement est en communication fluidique avec la poche de recueil par l'intermédiaire d'une première tubulure, ladite première tubulure comprenant un premier connecteur sur lequel est branchée l'extrémité amont d'une deuxième tubulure de sorte a être en communication fluidique avec la première tubulure, ladite deuxième tubulure comprénant une zone de pompage qui est agencée pour pouvoir être écrasée partiellement par la tête d'une pompe péristaltique de sorte à permettre l'alimentation sélective d'une part du dispositif d'échantillonnage en fluide prélevé et d'autre part de la poche de recueil en solution anticoagulante et/ou de conservation.

Ce remplissage manuel des tubes par l'utilisateur est une opération génératrice de temps pour l'utilisateur. En effet, l'utilisateur doit attendre qu'un tube soit rempli avant de commencer à remplir le suivant.

En outre, seule une partie du sang contenue dans la poche d'échantillonnage est utilisée pour la prise d'échantillon. Le sang restant dans la poche d'échantillonnage est perdu puisqu'il ne doit pas être envoyé dans la poche de recueil. Ainsi, on prélève le donneur d'une quantité de sang plus importante que nécessaire.

Enfin, un tel système à poches nécessite l'utilisation de tubes pressurisés ou sous vide qui subissent une perte de vide au cours du temps. Cette perte de vide entraîne un remplissage insuffisant du tube et peut fausser les analyses effectuées ultérieurement sur le sang contenu dans le tube.

L'invention a notamment pour but de résoudre ces problèmes en proposant un système à poches permettant d'automatiser et contrôler la prise d'échantillons lors d'un prélèvement d'un fluide biologique. En outre, le système utilise des tubes non pressurisés plus simple de fabrication et pouvant être conservés plus longtemps. Enfin, le système permet également de prélever par écoulement naturel le fluide biologique additionné de façon proportionnelle d'une solution anticoagulante et/ou de conservation.

A cet effet, et selon un premier aspect, l'invention propose un système à poches destiné au prélèvement et à l'échantillonnage d'un fluide biologique, notamment sanguin, du type comprenant un moyen de prélèvement dudit fluide, au moins une poche contenant une solution anticoagulante et/ou de conservation du fluide prélevé, au moins une poche de recueil destinée à recevoir le fluide prélevé additionné de la solution anticoagulante et/ou de conservation, et un dispositif d'échantillonnage du fluide prélevé, dans lequel le moyen de prélèvement est en communication fluidique avec la poche de recueil par l'intermédiaire d'une première tubulure, ladite première tubulure comprenant un premier connecteur sur lequel est branchée l'extrémité amont d'une deuxième tubulure de sorte à être en communication fluidique avec la première tubulure, l'extrémité aval de ladite deuxième tubulure étant pourvue d'un deuxième connecteur par l'intermédiaire duquel la poche contenant la solution et le dispositif d'échantillonnage sont en communication fluidique avec la deuxième tubulure au moyen respectivement d'une troisième et d'une quatrième tubulure branchées sur ledit deuxième connecteur, et la deuxième tubulure comprenant une zone de pompage qui est agencée pour pouvoir être écrasée partiellement par la tête d'une pompe péristaltique de sorte à permettre l'alimentation sélective d'une part du dispositif d'échantillonnage en fluide prélevé et d'autre part de la poche de recueil en solution anticoagulante et/ou de conservation.

Selon un deuxième aspect, l'invention propose un ensemble comprenant une pompe péristaltique et un système à poches selon le premier aspect de l'invention, la zone de pompage et la tête de la pompe étant agencées pour coopérer de sorte à permettre l'alimentation sélective d'une part du dispositif d'échantillonnage en fluide prélevé et d'autre part de la poche de recueil en solution anticoagulante et/ou de conservation.

D'autres objets et avantages apparaîtront au cours de la description qui suit en référence aux dessins annexés.
La figure 1 représente de façon schématique un ensemble comprenant un système à poches et une pompe péristaltique selon un premier mode de réalisation de l'invention.
Les figures 2A et 2B représentent de façon schématique deux modes de réalisation d'un tube muni d'un moyen de connexion à une tubulure d'un système à poches de l'invention.
La figure 3 représente de façon schématique un tube pourvu d'une sortie d'air selon l'invention.
Les figures 4 et 5 représentent de façon schématique un système à poches selon différents modes de réalisation de l'invention comprenant plusieurs tubes.
La figure 6 est une vue schématique en perspective d'une machine de mélange dans laquelle un système à poches selon l'invention est destiné à être utilisé.

L'invention concerne un système à poches pour le prélèvement et l'échantillonnage d'un fluide biologique, notamment sanguin.

Les termes "amont" et "aval" sont définis par rapport au sens d'écoulement du fluide biologique dans le système à poches, du moyen de prélèvement vers la poche de recueil et vers le dispositif d'échantillonnage.

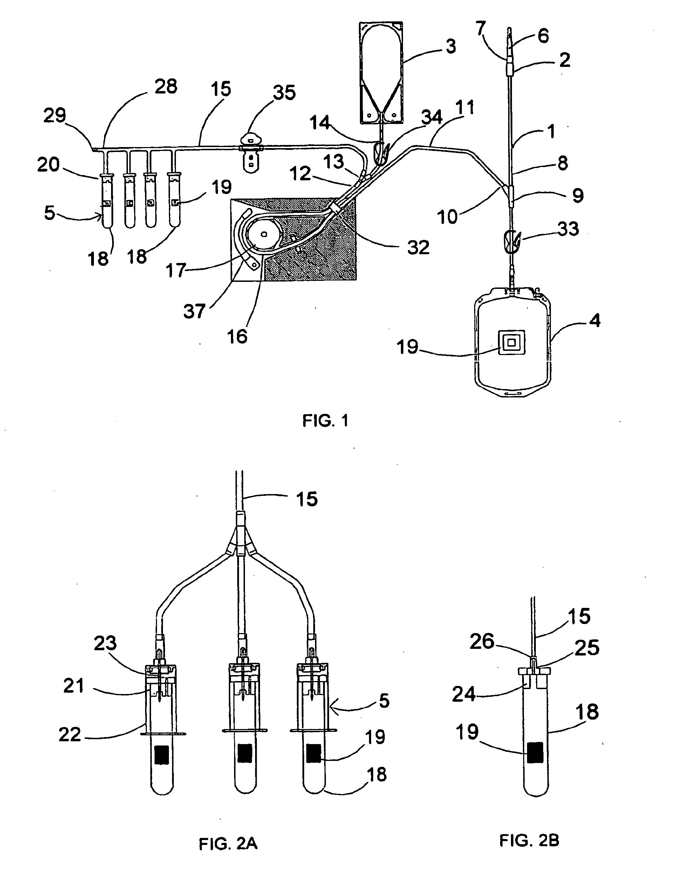

La figure 1 représente un système à poches 1 destiné au prélèvement et à l'échantillonnage d'un fluide biologique, notamment sanguin, du type comprenant un moyen de prélèvement 2 dudit fluide, au moins une poche 3 contenant une solution anticoagulante et/ou de conservation du fluide prélevé, au moins une poche de recueil 4 destinée à recevoir le fluide prélevé additionné de la solution anticoagulante et/ou de conservation, et un dispositif d'échantillonnage 5 du fluide prélevé.

Le moyen de prélèvement 2 est constitué notamment d'une aiguille 6 permettant l'accès à la veine d'un donneur et d'un capuchon 7 protégeant l'aiguille. En outre, un protecteur d'aiguille (non représenté) peut être placé de façon coulissante sur une première tubulure 8 mettant en communication la poche de recueil 4 et les moyens de prélèvement 2.

Lorsque le fluide biologique est du sang prélevé d'un donneur, la solution anticoagulante et/ou de conservation est par exemple une solution de type ACD (acide citrate dextrose) ou CPD (citrate phosphate dextrose).

Le moyen de prélèvement 2 est en communication fluidique avec la poche de recueil 4 par l'intermédiaire de la première tubulure 8, ladite première tubulure 8 comprenant un premier connecteur 9 sur lequel est branchée l'extrémité amont 10 d'une deuxième tubulure 11 de sorte à être en communication fluidique avec la première tubulure 8.

Selon l'invention, l'extrémité aval 12 de la deuxième tubulure 11 est pourvue d'un deuxième connecteur 13 par l'intermédiaire duquel la poche 3 contenant la solution et le dispositif d'échantillonnage 5 sont en communication fluidique avec la deuxième tubulure 11 au moyen respectivement d'une troisième et d'une quatrième tubulures 14,15 branchées sur ledit deuxième connecteur 13.

Selon le mode de réalisation de la figure 1, le premier et le second connecteurs 9,13 sont formés chacun d'une jonction trois voies en forme de Y.

La deuxième tubulure 11 comprend une zone de pompage 16 qui est agencée pour pouvoir être écrasée partiellement par la tête d'une pompe péristaltique 17 de sorte à permettre l'alimentation sélective d'une part du dispositif d'échantillonnage 5 en fluide prélevé et d'autre part de la poche de recueil 4 en solution anticoagulante et/ou de conservation.

Selon un mode de réalisation particulier, tous les éléments d'un tel système à poches 1 sont pré-connectés de sorte à former un système unitaire fermé ou en circuit clos. Le système ainsi formé est donc prêt-à-l'emploi.

Par exemple, le système à poches est stérilisé et/ou pasteurisé et conditionné dans un emballage stérile.

Les poches et les tubulures du système sont réalisées notamment en un matériau thermoplastique souple et stérilisable tel que le polychlorure de vinyle. Toutes les tubulures sont notamment souples, sécables et soudables.

Le dispositif d'échantillonnage est destiné à recevoir une partie du fluide prélevé. Dans le cas où le fluide biologique est du sang d'un donneur, cet échantillon de sang doit être analysé afin de vérifier l'absence d'agents pathogènes et de déterminer le groupe Rhésus.

Le dispositif d'échantillonnage 5 est en communication fluidique avec le moyen de prélèvement 2 par l'intermédiaire des quatrième, deuxième et première tubulures 15, 11, 8. Par conséquent, lors de l'échantillonnage, le dispositif d'échantillonnage 5 est directement alimenté en fluide prélevé à l'aide de la pompe péristaltique 17.

L'utilisation de la pompe péristaltique présente l'avantage de pouvoir contrôler la quantité de fluide prélevée, par exemple, en suivant le nombre de tours réalisés par la tête de pompe, et donc de ne prélever que la quantité de fluide nécessaire pour réaliser l'échantillon.

Le dispositif d'échantillonnage 5 est souple, rigide ou semi rigide. Selon un mode de réalisation, le dispositif d'échantillonnage comprend au moins un tube 18 destiné à contenir un échantillon du fluide, ledit tube étant en communication fluidique avec la quatrième tubulure.

Le tube étant en communication fluidique avec le moyen de prélèvement 2 par l'intermédiaire des quatrième, deuxième et première tubulures 15,11,8, il est alimenté directement et automatiquement en fluide prélevé par l'action de la pompe péristaltique 17. Il n'est donc plus nécessaire de prévoir une poche intermédiaire d'échantillonnage.

Le remplissage direct et automatique du tube est un gain de temps important. De plus, le tube contenant l'échantillon du fluide est compatible avec les automates d'analyse. Après dissociation du système à poches, il peut être directement placé dans un automate d'analyses, afin d'effectuer les analyses nécessaires.

En outre, le système à poches 1 étant en circuit clos, le tube 18 d'échantillonnage est connecté au système à poches 1 à l'origine, c'est-à-dire lors de la fabrication. Ainsi, il est possible d'identifier dès la fabrication par un marquage 19 telle qu'un code-barre ou une étiquette RFID, le tube 18 et la poche de recueil 4. Ce marquage 19 comprend des informations permettant, après dissociation du tube avec le système à poches d'établir de façon univoque que le tube d'échantillonnage et la poche de recueil proviennent du même système à poches.

La prise d'échantillon de sang est ainsi sécurisée et la traçabilité assurée. Le risque d'affecter un échantillon à une mauvaise poche de sang est nulle.

Afin d'établir la communication fluidique du tube 18 avec la quatrième tubulure 15, le tube 18 comprend un élément de fermeture 20 muni d'un moyen de connexion à la quatrième tubulure 15.

Selon la figure 2A, l'élément de fermeture est notamment un bouchon 21 en élastomère. Le moyen de connexion comprend un guide creux 22, ouvert en partie avant afin de permettre l'introduction et le retrait du tube 18, et une aiguille creuse 23 traversant la partie arrière du guide, de sorte qu'une partie aval de ladite aiguille s'étende à l'intérieur du guide 22 et traverse l'élément de fermeture et qu'une partie amont de ladite aiguille s'étende à l'extérieur du guide dans la quatrième tubulure 15.

La figure 2B représente un autre mode de connexion dans lequel l'élément de fermeture comprend un bouchon 24 munie d'une connexion 25 de type Luer mâle compatible avec une connexion 26 de type Luer femelle de la quatrième tubulure 15. A la fin de l'échantillonnage, le tube 18 est séparé du système à poches par simple déconnexion des connexions Luer mâle et femelle.

Le tube est notamment non pressurisé, c'est-à-dire qu'il n'est pas sous vide et contient de l'air. Les tubes non pressurisés sont plus faciles à fabriquer puisqu'il n'y a pas besoin de faire le vide d'air ni de garantir le maintien du vide jusqu'à leur utilisation.

Certains tubes sont pré-remplis à l'aide de réactifs ou d'anticoagulant. Si le vide n'est pas maintenu correctement dans le tube, la quantité d'échantillon de fluide dans le tube peut être insuffisante au regard de la quantité de réactif ou de solution anticoagulante, et les analyses peuvent être faussées.

En outre, si le système est clos et si le tube n'est pas assez souple, l'air contenu dans le tube doit être éliminé pour laisser la place au fluide prélevé.

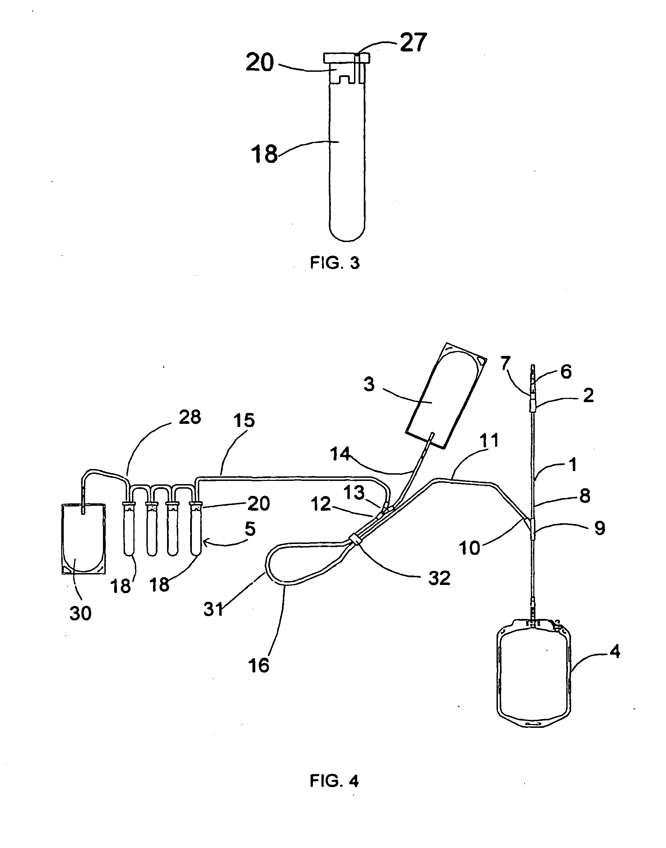

Le tube 18 est alors pourvu d'une sortie d'air 27 comprenant un filtre hydrophobe perméable aux gaz, comme illustré sur la figure 3. Le filtre hydrophobe empêche le passage de liquide tout en laissant passer les gaz. Tant que le fluide n'entre pas en contact avec le filtre hydrophobe, le tube se remplit de fluide prélevé et l'air initialement contenu dans le tube s'échappe par la sortie d'air. Lorsque le fluide entre en contact avec le filtre hydrophobe, le tube est plein.

En variante, le tube 18 est connecté à une cinquième tubulure 28 dont l'extrémité opposée audit tube comprend une sortie d'air 29 comprenant par exemple un filtre hydrophobe perméable aux gaz (Fig. 1).

Selon une autre variante, le tube 18 est connecté à une cinquième tubulure 28 dont l'extrémité opposée audit tube est connectée à une poche additionnelle 30 (Fig. 4).

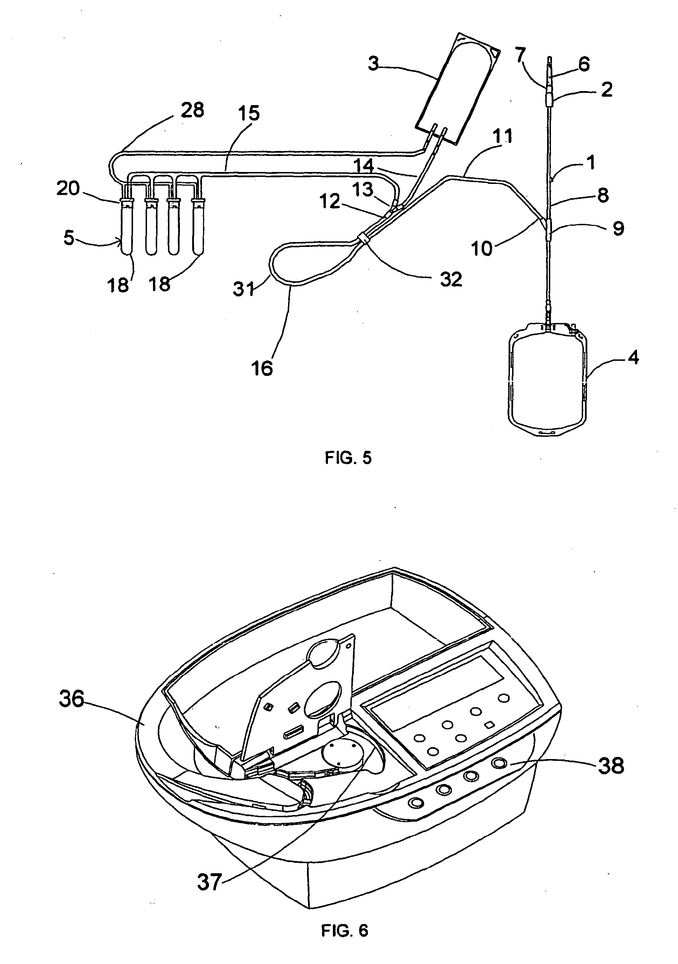

En variante représentée sur la figure 5, l'extrémité de la cinquième tubulure 28 est connectée à la poche 3 contenant la solution anticoagulante et/ou de conservation.

Dans ces deux dernières variantes, le système est en circuit clos. Il ne possède aucune ouverture sur l'extérieur et assure donc une très bonne stérilité. L'air initialement contenu dans le tube s'échappe dans la poche additionnelle 30 ou la poche 3 contenant la solution anticoagulante et/ou de conservation.

Lors d'un prélèvement sanguin, plusieurs tubes d'échantillonnage 18 doivent être remplis. Selon les législations nationales, le nombre de tubes à remplir varie entre 3 et 6. Comme représenté sur les figures 1, 4 et 5, le dispositif d'échantillonnage 5 du système à poches selon l'invention comprend alors plusieurs tubes 18 qui sont connectés en parallèle ou en série sur la quatrième tubulure 15.

Dans un mode de réalisation non représenté, la pluralité de tube est disposé sur un porte tube unique, de façon a faciliter leur manipulation, notamment après déconnexion du système à poches.

Selon l'invention, la deuxième tubulure 11 comprend une zone de pompage 16 qui est agencée pour pouvoir être écrasé partiellement par la tête d'une pompe péristaltique 17.

Comme représenté sur les figures 1, 4 et 5, la zone de pompage est agencée de sorte à pouvoir former une boucle 31 destinée à être enroulée autour de la tête de la pompe 17.

En particulier, la boucle est formée en utilisant une pièce telle que celle décrite dans le FR-A-2 850 581. Cette boucle préformée facilite le placement du système autour de la tête de pompe péristaltique.

Afin de contrôler l'écoulement des fluides dans le système à poches 1, la portion de la première tubulure située en aval du premier connecteur 9, la troisième et la quatrième tubulures 14,15 sont pourvues chacune d'un moyen permettant de fermer l'écoulement du fluide au travers d'elle.

Ce moyen est notamment un clamp 33,34,35, qui peut être manuel ou commandé par un dispositif de pilotage, réversible ou irréversible.

Dans un exemple particulier, le système à poches est destiné à la séparation et à la filtration des composants sanguins en circuit clos. Pour ce faire, le système à poches comprend un sous-système (non représenté) de traitement du fluide prélevé additionné d'une solution anticoagulante et/ou de conservation. Le sous-système est en communication fluidique avec la poche de recueil.

Le sous-système non représenté comprend une ou plusieurs poches satellites et au moins une unité de filtration capable notamment de retenir les leucocytes en circuit clos.

Selon un autre aspect représenté sur la figure 1, l'invention concerne un ensemble comprenant une pompe péristaltique 17 et un système à poches 1 tel qu'il vient d'être décrit. La zone de pompage 16 et la tête de la pompe 17 sont agencées pour coopérer de sorte à permettre l'alimentation sélective d'une part du dispositif d'échantillonnage 5 en fluide prélevé et d'autre part de la poche de recueil 4 en solution anticoagulante et/ou de conservation.

En particulier, la pompe 17 comprend un dispositif de pilotage qui est agencé pour commander la rotation sélective de la tête dans un premier et dans un deuxième sens, de sorte à alimenter le dispositif d'échantillonnage 5 en fluide lorsque la tête tourne dans le premier sens et la poche de recueil 4 en solution anticoagulante et/ou de conservation lorsque la tête tourne dans le deuxième sens.

Afin d'automatiser les étapes d'échantillonnage et de prélèvement, la pompe comprend un dispositif piloté de fermeture sélective de l'écoulement dans la portion de la première tubulure située en aval du premier connecteur 9 ainsi que dans les troisième et quatrième tubulures 14,15.

Notamment, un ensemble de clamps et de détecteurs positionnés de façon appropriée et piloté permettent de contrôler l'écoulement de fluide et de solution dans les tubulures du système à poches.

Ainsi, une seule pompe péristaltique 17 suffit à automatiser la prise d'échantillon et le prélèvement de fluide biologique. Après l'introduction du moyen de prélèvement 2 dans la source de fluide, les étapes successive d'échantillonnage et de prélèvement de fluide sont réalisées sans intervention humaine.

Dans un exemple particulier, on décrit maintenant la mise en oeuvre de l'ensemble représenté sur la figure 1, en relation avec un prélèvement de sang.

Initialement, les clamps 33,35 sur la première et la troisième tubulure 8,14 sont fermés, le clamp 34 sur la quatrième tubulure 15 est ouvert. On réalise une ponction veineuse d'un donneur. Puis, les tubes 18 du dispositif d'échantillonnage 5 sont alimentés en sang par rotation dans un premier sens de la tête de la pompe péristaltique 17.

Selon la figure 1, l'air présent dans les tubes est chassé à l'extérieur du système par la sortie d'air 29.

En mesurant le nombre de tours effectués par la tête de pompe, la quantité de sang échantillonnée dans les tubes peut être calculée de façon précise. Ainsi, il est possible de ne prélever que la quantité juste nécessaire aux analyses ultérieures et ainsi éviter de jeter du fluide qui serait en surplus.

Lorsque les tubes 18 sont remplis avec la quantité nécessaire de sang, le clamp 35 sur la quatrième tubulure 15 est fermé, et le clamp 34 sur la troisième tubulure 14 est ouvert. La tête de pompe effectue alors des rotations dans le deuxième sens, de sorte à alimenter la poche de recueil 4 en solution anticoagulante et/ou de conservation. Le clamp 33 sur la première tubulure 8 étant ouvert, le sang du donneur s'écoule également par gravité dans la poche de recueil 4.

Pendant ou après cette étape de prélèvement, les tubes 18 sont dissociés du système à poches, par déconnexion de la quatrième tubulure 15. Cette déconnexion s'effectue par exemple par soudage à chaud de la quatrième tubulure, juste au dessus de l'élément de fermeture 20 du tube 18.

En variante, il est également possible d'effectuer d'abord le remplissage de la poche de recueil 4 avec le fluide le biologique, puis le remplissage du dispositif d'échantillonnage 5.

Comme la pompe comprend des dispositifs de pilotage pour contrôler la rotation de la tête de pompe et l'écoulement des fluides dans les différentes tubulures, l'échantillonnage et le prélèvement sont complètement automatisés, engendrant un gain de temps pour le personnel chargé du don du sang.

Dans un exemple particulier, la pompe 17 est intégrée dans une machine de mélange en continu et selon un ratio déterminé du fluide et de la solution dans la poche de recueil 4, ladite machine comprenant un dispositif de mesure du volume de fluide prélevé par écoulement naturel et un dispositif de pilotage de la vitesse de pompage de la solution en fonction du volume prélevé.

Une telle machine 36 est représentée sur la figure 6 et décrite dans le FR-A-285 0561. La machine représentée permet à la solution anticoagulante d'être ajoutée de façon proportionnelle au sang prélevé par écoulement naturel. Un ratio entre la quantité de solution anticoagulante et/ou de conservation est notamment de 1/7.

Comme représenté sur la figure 6, la machine de mélange 36 comprend un support 37 dans lequel la zone de pompage 16 est immobilisée en regard de la tête de la pompe 17 de sorte à être écrasée partiellement lors de la rotation de celle-ci.

En outre, la machine 36 comprend un support 38 pour tubes de sorte à positionner les tubes verticalement.

## Revendications

1. Système à poches (1) destiné au prélèvement et à l'échantillonnage d'un fluide biologique, notamment sanguin, comprenant un moyen de prélèvement (2) dudit fluide, au moins une poche (3) contenant une solution anticoagulante et/ou de conservation du fluide prélevé, au moins une poche de recueil (4) destinée à recevoir le fluide prélevé additionné de la solution anticoagulante et/ou de conservation, et un dispositif d'échantillonnage (5) du fluide prélevé, dans lequel le moyen de prélèvement (2) est en communication fluidique avec la poche de recueil (4) par l'intermédiaire d'une première tubulure (8), ladite première tubulure comprenant un premier connecteur (9) sur lequel est branchée l'extrémité amont (10) d'une deuxième tubulure (11) de sorte à être en communication fluidique avec la première tubulure (8), où l'extrémité aval (12) de ladite deuxième tubulure (11) est pourvue d'un deuxième connecteur (13) par l'intermédiaire duquel la poche (3) contenant la solution et le dispositif d'échantillonnage (5) sont en communication fluidique avec la deuxième tubulure (11) au moyen respectivement d'une troisième et d'une quatrième tubulures (14,15) branchées sur ledit deuxième connecteur (13), et ladite deuxième tubulure (11) comprend une zone de pompage (16) qui est agencée pour pouvoir être écrasée partiellement par la tête d'une pompe péristaltique (17) de sorte à permettre l'alimentation sélective d'une part du dispositif d'échantillonnage (5) en fluide prélevé et d'autre part de la poche de recueil (4) en solution anticoagulante et/ou de conservation.

2. Système à poches selon la revendication 1, **caractérisé en ce que** le dispositif d'échantillonnage (5) comprend au moins un tube (18) destiné à contenir un échantillon du fluide, ledit tube étant en communication fluidique avec la quatrième tubulure (15).

3. Système à poches selon la revendication 2, **caractérisé en ce que** le tube (18) comprend un élément de fermeture (20) muni d'un moyen de connexion à la quatrième tubulure (15).

4. Système à poches selon la revendication 2 ou 3, **caractérisé en ce que** le tube (18) est non pressurisé.

5. Système à poches selon la revendication 4, **caractérisé en ce que** le tube (18) est pourvu d'une sortie d'air (27) comprenant un filtre hydrophobe perméable aux gaz.

6. Système à poches selon la revendication 4, **caractérisé en ce que** le tube (18) est connecté à une cinquième tubulure (28) dont l'extrémité opposée audit tube comprend une sortie d'air (29) ou est connectée à une poche (30).

7. Système à poches selon la revendication 6, **caractérisé en ce que** l'extrémité de la cinquième tubulure (28) est connectée à la poche (3) contenant la solution anticoagulante et/ou de conservation.

8. Système à poches selon l'une quelconque des revendications 2 à 7, **caractérisé en ce que** le dispositif d'échantillonnage (5) comprend plusieurs tubes (18) qui sont connectés en parallèle ou en série sur la quatrième tubulure (15).

9. Système à poches selon l'une quelconque des revendications 1 à 8, **caractérisé en ce que** la zone de pompage (16) est agencée de sorte à pouvoir former une boucle (31) destinée à être enroulée autour de la tête de la pompe (17).

10. Système à poches selon l'une quelconque des revendications 1 à 9, **caractérisé en ce que** la portion de la première tubulure (8) située en aval du premier connecteur (9), la troisième et la quatrième tubulures (14,15) sont pourvues chacune d'un moyen permettant de fermer l'écoulement du fluide au travers d'elle.

11. Ensemble comprenant une pompe péristaltique (17) et un système à poches (1) selon l'une quelconque des revendications 1 à 10, la zone de pompage (16) et la tête de la pompe (17) étant agencées pour coopérer de sorte à permettre l'alimentation sélective d'une part du dispositif d'échantillonnage (5) en fluide prélevé et d'autre part de la poche de recueil (4) en solution anticoagulante et/ou de conservation.

12. Ensemble selon la revendication 11, **caractérisé en ce que** la pompe (17) comprend un dispositif de pilotage qui est agencé pour commander la rotation sélective de la tête dans un premier et dans un deuxième sens, de sorte à alimenter le dispositif d'échantillonnage (5) en fluide lorsque la tête tourne dans le premier sens et la poche de recueil (4) en solution lorsque la tête tourne dans le deuxième sens.

13. Ensemble selon la revendication 11 ou 12, **caractérisé en ce que** la pompe (17) comprend un dispositif piloté de fermeture sélective de l'écoulement dans la portion de la première tubulure (8) située en aval du premier connecteur (9) ainsi que dans les troisième et quatrième tubulures (14,15).

14. Ensemble selon l'une quelconque des revendications 11 à 13, **caractérisé en ce que** la pompe (17) est intégrée dans une machine (36) de mélange en continu et selon un ratio déterminé du fluide et de la solution dans la poche de recueil (4), ladite machine comprenant un dispositif de mesure du volume de fluide prélevé par écoulement naturel et un dispositif de pilotage de la vitesse de pompage de la solution en fonction du volume prélevé.

15. Ensemble selon la revendication 14, **caractérisé en ce que** la machine (36) de mélange comprend un support (37) dans lequel la zone de pompage est immobilisée en regard de la tête de la pompe (17) de sorte à être écrasée partiellement lors de la rotation de celle-ci.

## Claims

1. A system of bags (1) intended for the sampling and the collection of a biological fluid, more particularly blood, including means (2) for sampling said fluid, at least one bag (3) containing an anticoagulant and/or sampled fluid preservation solution, at least one collection bag (4) intended for receiving the sampled fluid to which the anticoagulant and/or preservation solution has been added, and means (5) for sampling the collected fluid, wherein the sampling means (2) is in fluidic communication with the collection bag (4) through a first tubing (8), said first tubing including a first connector (9) which the upstream end (10) of a second tubing (11) is connected to, so as to be in fluidic communication with the first tubing (8), where the downstream end (12) of said second tubing (11) is provided with a second connector (13) through which the bag (3) containing the solution and the sampling device (5) are in fluidic communication with the second tubing (11) through a third and a fourth tubing (14, 15), respectively, connected to said second connector (13), and said second tubing (11) includes a pumping zone (16) which is so arranged as to be in a position to be partially crushed by the head of a peristaltic pump (17) so as to enable the selective supply on the one hand of the sampling device (5) with the collected fluid and on the other hand of the anticoagulant and/or preservation solution collection bag (4).

2. A system of bags according to claim 1, **characterized in that** the sampling device (5) includes at least one tube (18) intended to contain a sample of the fluid, said tube being in fluidic communication with the fourth tubing (15).

3. A system of bags according to claim 2, **characterized in that** the tube (18) includes a closing element (20) provided with means for the connection to the fourth tubing (15).

4. A system of bags according to claim 2 or 3,
**characterized in that** the tube (18) is not pressurized.

5. A system of bags according to claim 4, **characterized in that** the tube (18) is provided with an air outlet (27) including a gas permeable hydrophobic filter.

6. A system of bags according to claim 4, **characterized in that** the tube (18) is connected to a fifth tubing (28), the end opposite said tube of which includes an air outlet (29) or is connected to a bag (30).

7. A system of bags according to claim 6, **characterized in that** the end of the fifth tubing (28) is connected to the bag (3) containing the anticoagulant and/or preservation solution.

8. A system of bags according to any one of claims 2 to 7, **characterized in that** the sampling device (5) includes several tubes (18) which are connected in parallel or in series on the fourth tubing (15).

9. A system of bags according to any one of claims 1 to 8, **characterized in that** the pumping zone (16) is so arranged as to form a loop (31) intended to be wound about the pumping head (17).

10. A system of bags according to any one of claims 1 to 9, **characterized in that** the portion of the first tubing (8) located downstream of the first connector (9), the third and the fourth tubings (14, 15) are each provided with means enabling to close the flowing of the fluid there through.

11. An assembly including a peristaltic pump (17) and a system of bags (1) according to any one of claims 1 to 10, the pumping area (16) and the pump head (17) being so arranged for cooperating so as to enable the selective supply, on the one hand, of the collected fluid sampling device (5) and, on the other hand the anticoagulant and/or preservation solution collection bag (4).

12. An assembly according to claim 11, **characterized in that** the pump (17) includes a controlling device which is so arranged as to control the selective rotation of the head in a first and in a second direction, so as to supply the sampling device (5) with fluid when the head rotates in the first direction and the solution collection bag (4) when the head rotates in the second direction.

13. An assembly according to claim 11 or 12, **characterized in that** the pump (17) includes a controlled device for selectively closing the flow in the portion of the first tubing (8) positioned downstream of the first connector (9) as well as in the third and fourth tubings (14, 15).

14. An assembly according to any one of claims 11 to 13, **characterized in that** the pump (17) is integrated in a continuous mixing machine (36) and according to a determined ratio of the fluid and the solution in the collection bag (4), said machine including a device for measuring the volume of the fluid sampled by natural flow and a device for controlling the solution pumping speed as a function of the sampled volume.

15. An assembly according to claim 14, **characterized in that** the mixing machine (36) includes a support (37) wherein the pumping zone is blocked opposite the pump head (17) so as to be partially crushed during the rotation thereof.

## Patentansprüche

1. Beutelsystem (1), das zur Entnahme und zur Probennahme einer biologischen Flüssigkeit, insbesondere Blut, bestimmt ist, umfassend ein Entnahmemittel (2) der genannten Flüssigkeit, wenigstens einen Beutel (3), der eine antikoagulierende und / oder konservierende Lösung der entnommenen Flüssigkeit umfasst, wenigstens einen Auffangbeutel (4), der zur Aufnahme der entnommenen, der antikoagulierenden und / oder konservierenden Lösung hinzugefügten Flüssigkeit bestimmt, und eine Probennahmevorrichtung (5) der entnommenen Flüssigkeit, in der das Entnahmemittel (2) in flüssiger Kommunikation mit dem Auffangbeutel (4) über ein erstes Röhrchen (8) ist, wobei das genannte erste Röhrchen ein erstes Verbindungsstück (9) umfasst, an das das obere Ende (10) eines zweiten Röhrchens (11) derart angeschlossen ist, dass es in flüssiger Kommunikation mit dem ersten Röhrchen (8) steht, in dem das untere Ende (12) des genannten zweiten Röhrchens (11) mit einem zweiten Verbindungsstück (13) versehen ist, über das der Beutel (3), der die Lösung und die Probennahmevorrichtung (5) enthält, in flüssiger Kommunikation mit dem zweiten Röhrchen (11) jeweils mit einem dritten und einem vierten Röhrchen (14, 15) stehen, die an dem genannten zweiten Verbindungsstück (13) angeschlossen sind, und das genannte zweite Röhrchen (11) eine Pumpenzone (16) umfasst, die angeordnet ist, um teilweise von dem Kopf einer peristaltischen Pumpe (17) derart flach gedrückt zu werden, dass die selektive Versorgung einerseits der Probennahmevorrichtung (5) mit entnommener Flüssigkeit und andererseits des Auffangbeutels (4) mit antikoagulierender und / oder konservierender Lösung erlaubt wird.

2. Beutelsystem gemäß Anspruch 1, **dadurch gekennzeichnet, dass** die Probennahmevorrichtung (5) wenigstens eine Röhre (18) umfasst, die dazu bestimmt ist, eine Probe der Flüssigkeit zu enthalten, wobei die genannte Röhre mit dem vierten Röhrchen (15) in flüssiger Kommunikation steht.

3. Beutelsystem gemäß Anspruch 2, **dadurch gekennzeichnet, dass** die Röhre (18) ein Verschlusselement (20) umfasst, das mit einem Verbindungsmittel mit dem vierten Röhrchen (15) versehen ist.

4. Beutelsystem gemäß Anspruch 2 oder 3, **dadurch gekennzeichnet, dass** die Röhre (18) nicht unter Druck steht.

5. Beutelsystem gemäß Anspruch 4, **dadurch gekennzeichnet, dass** die Röhre (18) mit einem Luftausgang (27) versehen ist, der einen hydrophoben, gasdurchlässigen Filter umfasst.

6. Beutelsystem gemäß Anspruch 4, **dadurch gekennzeichnet, dass** die Röhre (18) an ein fünftes Röhrchen (28) angeschlossen ist, dessen der genannten Röhre gegenüberliegendes Ende einen Luftausgang (29) umfasst oder an einen Beutel (30) angeschlossen ist.

7. Beutelsystem gemäß Anspruch 6, **dadurch gekennzeichnet, dass** das Ende des fünften Röhrchens (28) an den Beutel (3) angeschlossen ist, der die antikoagulierende und / oder konservierende Lösung enthält.

8. Beutelsystem gemäß Anspruch 2 bis 7, **dadurch gekennzeichnet, dass** die Probennahmevorrichtung (5) mehrere Röhren (18) umfasst, die parallel oder in Serie an das vierte Röhrchen (15) angeschlossen sind.

9. Beutelsystem gemäß Anspruch 1 bis 8, **dadurch gekennzeichnet, dass** die Pumpzone (16) derart angeordnet ist, dass eine Schleife (31) gebildet werden kann, die dazu bestimmt ist, um den Kopf der Pumpe (17) aufgerollt zu werden.

10. Beutelsystem gemäß Anspruch 1 bis 9, **dadurch gekennzeichnet, dass** der Abschnitt des ersten Röhrchens (8) sich unterhalb des ersten Verbindungsstücks (9) befindet, das dritte und vierte Röhrchen (14, 15) jeweils mit einem Mittel versehen sind, das den Verschluss des Abflusses der Flüssigkeit durch diesen erlaubt.

11. Struktur mit einer peristaltischen Pumpe (17) und einem Beutelsystem (1) gemäß Anspruch 1 bis 10, wobei die Pumpzone (16) und der Kopf der Pumpe (17) angeordnet sind, um derart zusammenzuwirken, dass die selektive Versorgung einerseits der Probennahmevorrichtung (5) mit entnommener Flüssigkeit und andererseits des Auffangbeutels (4) mit antikoagulierender und / oder konservierender Lösung erlaubt wird.

12. Struktur gemäß Anspruch 11, **dadurch gekennzeichnet, dass** die Pumpe (17) eine Steuervorrichtung umfasst, die angeordnet ist, um die selektive Rotation des Kopfes in einer ersten und einer zweiten Richtung derart zu erlauben, dass die Probennahmevorrichtung (5) mit Flüssigkeit versorgt wird, wenn der Kopf sich in der ersten Richtung dreht und der Auffangbeutel (4) mit Flüssigkeit, wenn der Kopf sich in der zweiten Richtung dreht.

13. Struktur gemäß Anspruch 11 oder 12, **dadurch gekennzeichnet, dass** die Pumpe (17) eine gesteuerte Vorrichtung zum selektiven Verschluss des Abflusses in dem Abschnitt des ersten Röhrchens (8) umfasst, das sich unterhalb des ersten Verbindungsstücks (9) befindet, sowie in dem dritten und vierten Röhrchen (14, 15).

14. Struktur gemäß Anspruch 11 bis 13, **dadurch gekennzeichnet, dass** die Pumpe (17) in eine Maschine (36) zum kontinuierlichen und zum Mischen gemäß einem bestimmten Verhältnis der Flüssigkeit und der Lösung in dem Auffangbeutel (14) integriert ist, wobei die genannte Maschine eine Messvorrichtung des Volumens der entnommenen Flüssigkeit durch natürliches Abfließen und eine Steuervorrichtung der Pumpgeschwindigkeit der Lösung in Abhängigkeit des entnommenen Volumens umfasst.

15. Struktur gemäß Anspruch 4, **dadurch gekennzeichnet, dass** die Maschine (36) zum Mischen einen Träger (37) umfasst, in dem die Pumpzone gegenüber dem Kopf der Pumpe (17) derart blockiert ist, dass sie teilweise bei der Rotation derselben flach gedrückt wird.
